# EUROPÄISCHE PATENTSCHRIFT

(11) **EP 2 296 561 B1**
(45) Veröffentlichungstag und Bekanntmachung des Hinweises auf die Patenterteilung: **15.02.2012**
(21) Anmeldenummer: 09761748.4
(22) Anmeldetag: 10.06.2009
(51) Int. Cl.: A61B 17/10

(54) **ENDOSKOPKAPPE**
ENDOSCOPE CAP
BOUCHON POUR ENDOSCOPE

(30) Priorität: 11.06.2008 DE 202008007774 U
(43) Veröffentlichungstag der Anmeldung: 23.03.2011
(73) Patentinhaber: Ovesco Endoscopy AG, 72074 Tübingen (DE)
(72) Erfinder: HO, Chi-Nghia, 70180 Stuttgart (DE); ANHÖCK, Gunnar, 72762 Reutlingen (DE); SCHURR, Marc. O., 72072 Tübingen (DE)
(74) Vertreter: Winter, Brandl, Fürniss, Hübner, Röss, Kaiser, Polte - Partnerschaft
(86) Internationale Anmeldenummer: PCT/EP2009/057210
(87) Internationale Veröffentlichungsnummer: WO 2009/150186

(56) Entgegenhaltungen:
- WO-A-99/56635
- US-A- 5 320 630
- US-B2- 6 849 078

## Beschreibung

Die vorliegende Erfindung betrifft eine Endoskopkappe zur Platzierung von Gewebeclips vorzugsweise in Hohlorganen eines menschlichen oder tierischen Körpers.

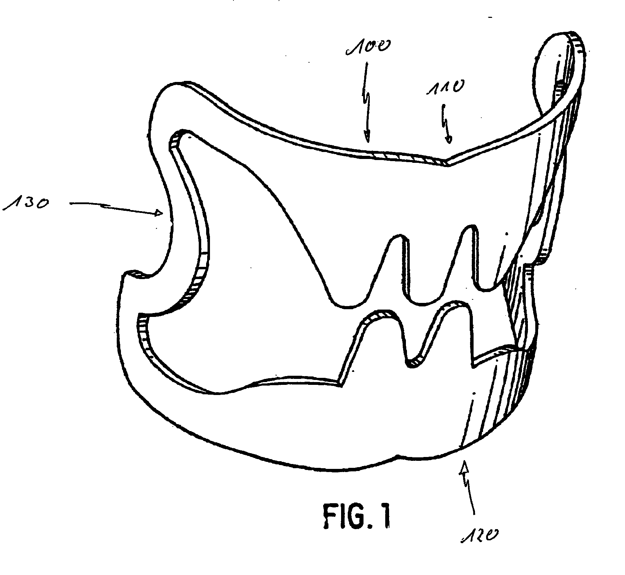

Aus dem Stand der Technik beispielsweise gemäß der US 6,849,078 B2 ist ein Gewebeclip dieser Gattung hinsichtlich seiner grundsätzlichen Konstruktion allgemein bekannt. Zum besseren Verständnis wird dieser nachstehend unter Verweis auf die Fig. 1 näher beschrieben.

Demzufolge besteht ein solcher Clip 100 aus einer maulartigen Klemmeinrichtung mit zwei gezahnten Kiefern 110, 120, welche über zwei seitliche Scharniere 130 bzw. durch flexible Ausformungen auf- und zugeklappt werden können. Die Scharniere 130 bzw. die flexiblen Ausformungen sind dabei vorzugsweise aus federelastischen Bändern ausgebildet, welche beim Aufklappen der Kiefer 110, 120 eine Federenergie speichern, die beim Freigeben der Kiefer 110, 120 d.h. bei einem Auslösen der Scharniere 130 bzw. der flexiblen Ausformungen zu einem Zuschnappen der Kiefer 110, 120 mit vorbestimmter Klemmkraft führt.

Im Einzelnen ist jeder Clip 100 einstückig aus einem Federblech gestanzt, indem aus dem Federblech ein Ring mit partiell unterschiedlicher Ringbreite herausgearbeitet wird. Zwei diametrisch gegenüberliegende Ringabschnitte mit großer Ringbreite bilden die beiden Kiefer 110, 120, wohingegen die beiden dazwischen liegenden Ringabschnitte mit schmaler Ringbreite die Scharniere 130 bzw. die flexiblen Ausformungen ergeben. Die Kiefer 110, 120 sind dadurch ausgebildet, indem die Ringabschnitte mit großer Ringbreite bogenförmig gewölbt werden, wobei die beiden Ringabschnitte mit schmaler Ringbreite um ihre Längsachse um ca. 180° verdreht (tordiert) ausgebildet sind, um die Scharniere auszuformen. Durch diese besondere Formgebung des ausgestanzten Federblechs entsteht die Form einer Art Haifischmaul mit zwei aufeinander sich zu bewegenden Zahnreihen, welche durch Ausstanzen der Ringabschnitte mit großer Ringbreite gebildet werden.

Die Funktionsweise des vorstehend beschriebenen medizinischen Gewebeclips 100 lässt sich wie folgt beschreiben:

Im Allgemeinen stellt eine endoskopische Implantation einer medizinischen Vorrichtung insgesamt das für den Patienten verträglichste Verfahren dar. In diesem Fall muss die medizinische Vorrichtung von der Innenseite eines Hohlorgans an diesem fixiert werden. Zu diesem Zwecke werden eine Anzahl der vorstehend beschriebenen Gewebeklammern, Clips oder Anker mittels eines Endoskops in das Hohlorgan eingeführt und an vorbestimmten Stellen an der Organinnenseite platziert. Hiefür wird der jeweilige Clip oder Anker an das Organgewebe herangeführt und die Vorspannfeder für ein Zuschnappen des Clips oder Aufspannen des Ankers ausgelöst. Dieser klemmt oder hält daraufhin eine Gewebefalte zwischen seinen Kiefern oder seinen Haken oder Nadeln mit einer vorbestimmten Klemm- oder Spreizkraft ein, wobei sich die Zähne, Haken, Nadeln oder Zacken jedes Kiefers in das Gewebe bohren und dieses vorzugsweise durchdringen. Auf diese Weise wird jeder Clip oder Anker in bestimmten Abständen zueinander an der Organinnenseite verankert und bildet somit für eine Zugkraft einen Einleitpunkt in das Organgewebe.

Das in der Fig. 1 nicht weiter dargestellte Endoskop ist in der Regel mit einem Endoskopkopf oder einer Endoskopkappe ausgerüstet, welche neben den für ein Endoskop generell erforderlichen Funktionen wie Beleuchtung, Optik und ggf. Spülvorrichtung zusätzlich eine Halte- und Abzieheinrichtung für den Gewebeclip aufweist. Diese Halte- und Abzieheinrichtung besteht im Wesentlichen aus einer Spreizhülse sowie einem manuell oder ferngesteuert betätigbaren Schieber, der in Endoskoplängsrichtung bewegbar ist. Die Spreizhülse ist dabei derart ausgebildet, dass der bereits geöffnete Gewebeclip auf die Hülse aufgesetzt werden kann, derart, dass ein nach hinten Rutschen des Clips während dessen Einführung in das Hohlorgan verhinderbar ist. Zu diesem Zweck ist der Schieber axial hinter dem Clip positioniert und dient quasi als Axialanschlag für den Clip.

Sobald der Clip an einer bestimmten Stelle platziert werden soll, wird der Schieber axial nach vorne bewegt und streift dabei den Clip über die Spreizhülse ab. Dabei wird der Clip ausgelöst, d.h. der vorstehend anhand der Fig. 1 beschriebene Vorspannmechanismus innerhalb des Clips wird beim Abstreifen von der Spreizhülse freigegeben und die beiden Kiefer des Gewebeclips schnappen unter Einklemmen des dazwischen liegenden Gewebes zu.

Bei Endoskopen dieser Gattung ist der Endoskopdurchmesser für deren Funktionsfähigkeit von entscheidender Bedeutung. Insofern besteht das grundsätzliche Problem, dass die Endoskopkappe mit allen notwendigen Funktionen ein großes Volumen hat und daher der Einsatzbereich eines solchen Endoskops eingeschränkt ist.

Angesichts dieser Problematik besteht die Aufgabe der vorliegenden Erfindung darin, eine Endoskopkappe für das Platzieren eines bereits bekannten Gewebeclips bereitzustellen, die möglichst klein baut.

Diese Aufgabe wird durch eine Endoskopkappe gelöst, welche die technischen Merkmale gemäß dem Patentanspruch 1 aufweist.

Der Kern der Erfindung besteht demzufolge in der Ausbildung einer in Umfangsrichtung beidseitig geöffneten Stirnnut am Außenumfang der Spreizhülse zur Aufnahme eines Gewebeclips, durch die ein Faden oder Gewebe in Radialrichtung gezogen ist. Der Faden ist an einem Ende am Endoskop bzw. an der Endoskopkappe fixiert und am anderen Ende längs des Endoskops bewegbar geführt, sodass der Faden beim Einschieben des Gewebeclips in die Nut von diesem mitgenommen werden kann. Wird der Faden anschließend gezogen, zeigt dieser die Tendenz, sich innerhalb der Stirnnut zu verkürzen, wobei der Gewebeclip vom Faden wieder aus der Nut geschoben wird.

Hierdurch wird folglich eine funktionsbezogene konstruktive Aufteilung der erfindungsgemäßen Halte- und Abzieheinrichtung erreicht, nämlich in die Anordnung der einen Axialanschlag ausbildenden Stirnnut/ -schlitz sowie in die hierzu separate Anordnung eines einen Schieber simulierenden Fadens. Diese konstruktive Zweiteilung der Halte- und Abzieheinrichtung ermöglicht es, insbesondere die Abzieheinrichtung als hochflexiblen Faden oder Kabel auszubilden, der nur wenig Bauraum benötigt und trotzdem ausreichend große Verschiebekräfte insbesondere bei Anwendung des vorstehend beschriebenen Flaschenzugmechanismus auf den Clip ausüben kann.

Weitere vorteilhafte Ausgestaltungen der Erfindung sind Gegenstand der Unteransprüche.

Die Erfindung wird nachstehend anhand eines bevorzugten Ausführungsbeispiels unter Bezugnahme auf die begleitenden Zeichnungen näher erläutert.

Es zeigen
Fig. 1 die beispielhafte Konstruktion eines Gewebeclips, wie er bereits aus dem Stand der Technik bekannt ist und wie er bei der vorliegenden Erfindung ebenfalls verwendet wird,
Fig. 2 den Längsschnitt einer Endoskopkappe gemäß der Erfindung mit aufgesetztem Gewebeclip gemäß Fig. 1 in schematischer Darstellung,
Fig. 3 den Längsschnitt aus Fig. 2 mit betätigter Halte- und Abzieheinrichtung gemäß der Erfindung,
Fig. 4 die schrittweise Funktionsdarstellung der erfindungsgemäßen Halte- und Abzieheinrichtung während der Betätigungsphase und
Fig. 5a-5c eine Variante für die Bereitstellung einer Clipführenden Stirnnut an der Kappenumfangswand.

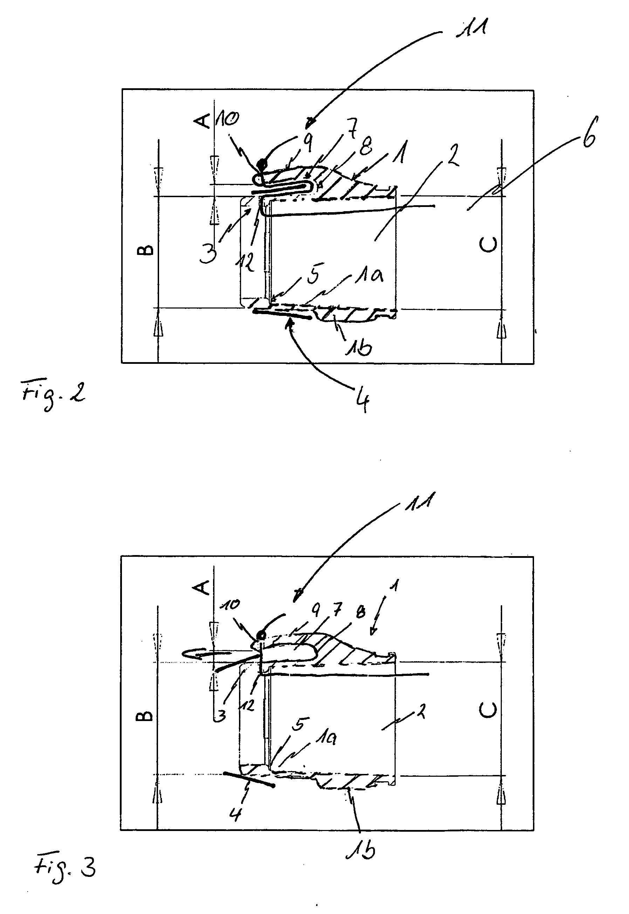

In der Fig. 2 ist eine Endoskopkappe 1 gemäß einem bevorzugten Ausführungsbeispiel der Erfindung am distalen Ende eines Endoskops oder Trokars angeordnet, welches in ein Hohlorgan eines menschlichen oder tierischen Körpers einführbar ist.

Die Endoskopkappe 1 gemäß der Erfindung hat einen Aufsteckabschnitt 1a, der im montierten Zustand einen distalen Endoskopkopf 2 umgibt, welcher wahlweise mit endoskopspezifischen Funktionen, beispielsweise Beleuchtung, Optik, Spüleinrichtung, Arbeitskanalmündung etc. ausgestattet ist, die von einer am proximalen Ende des Endoskops befindlichen Handhabe individuell bedienbar sind. Die Endoskopkappe 1 ist im axialabstand zum Aufsteckabschnitt 1a mantelseitig zu oder mit einer Spreizhülse 3 ausgeformt, auf die ein Gewebeclip 4 aufschiebbar ist, wie er vorstehend anhand der Fig. 1 näher beschrieben wurde. Diese Spreizhülse 3 steht dabei über die distale Stirnseite des Endoskopkopfs 2 axial vor und bildet somit einen an ihrer vorderen Kante radial nach Außen abgerundeten freien Ringabschnitt. Zur exakten axialen Positionierung der Endoskopkappe 1 hat diese eine radial innere Umfangskante 5, die sich an die Stirnseite des Endoskopkopfs 2 andrückt und somit ein Verschieben der Kappe 1 längs des Endoskops in Richtung zu dessen proximalen Ende unterbindet.

Im vorliegenden bevorzugten Ausführungsbeispiel bildet die Endoskopkappe 1 gleichzeitig auch das Gehäuse für den Endoskopkopf 2 und ist demzufolge als Bestandteil des Endoskops fest und dichtend mit einem Endoskopschaft 6 verbunden, welcher in der Fig. 2 lediglich andeutungsweise dargestellt ist. Alternativ hierzu kann die Endoskopkappe 1 mit dem gleichen konstruktiven Aufbau jedoch auch als ein zum Endoskopkopf 2 separates Bauteil gefertigt sein, welches auf ein dann bereits vorhandenes Gehäuse des Endoskopkopfs 2 mit den entsprechenden Funktionen auf- oder angesetzt werden kann und daher als Nachrüstsatz handelsüblicher Endoskope geeignet ist.

Die Endoskopkappe 1 gemäß der Erfindung weist in jedem Fall eine von der Stirnseite aus eingebrachte Stirnnut 7 in der mantelseitigen Kappenwand auf, welche sich vorzugsweise als teilkreis- bzw. sichelförmiger Schlitz an der distalen Stirnseite des Endoskopkopfs 2 bzw. der Kappe 1 öffnet und deren Nutengrund an einer axial hinteren Stelle, vorzugsweise etwa in einem axial mittleren Abschnitt des Endoskopkopfes 2 (im axial mittleren Bereich des Aufsteckabschnitts 1a) einen Anschlag 8 bildet. Der Radius der Stirnnut 7 ist jedoch größer gewählt als der Radius der Endoskopkappe 1, sodass die Kappenwand bei der Ausbildung der Stirnnut 7 zwei in Umfangsrichtung entsprechend beabstandete Schlitze erhält. Durch Ausbilden dieser Stirnnutschlitze wird die Kappenmantelwand in diesem Bereich somit längsgespalten, wodurch an der Außenseite der Kappenwand eine Art Lasche oder Zunge 9 entsteht, welche die radial äußere Nutenwand definiert.

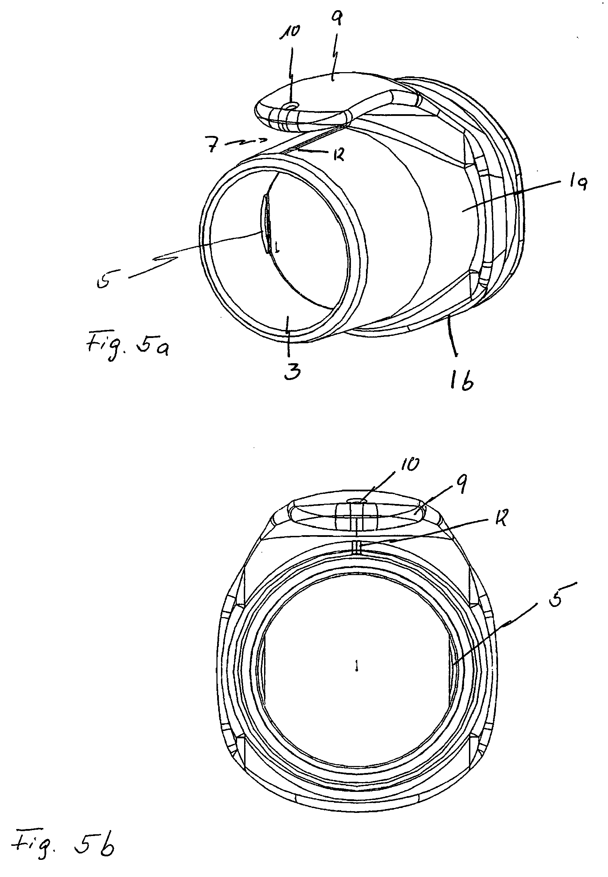

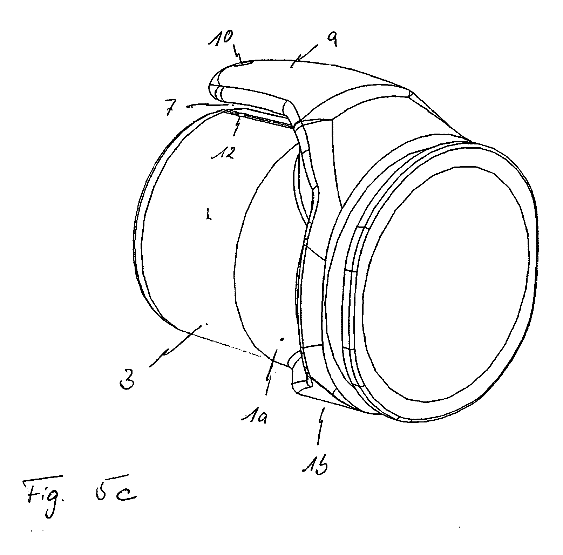

Eine weitere Variante der Bereitstellung einer Stirnnut gemäß vorstehender Definition ist gemäß der Fig. 5a-5c die zusätzliche Anordnung einer vorzugsweise in Axialrichtung gekrümmten Lasche oder Zunge 9, deren Wurzel einstückig mit der Kappe 1 im Bereich des Aufsteckabschnitts 1a ausgebildet ist und die sich unter Ausbildung der Nut 7 im Radialabstand zur Kappenmantelwand axial in Richtung Spreizhülse 3 erstreckt. In diesem Fall wird also die Mantelwand nicht gespalten (wie vorstehend beschrieben) sondern es wird ein zusätzliches Bauteil in Form der Lasche 9 über die Mantelwand der Kappe 1 geführt. Diese Lasche 9 kann derart schmal dimensioniert sein, dass sie im Querschnitt geradlinig (ohne Radius) bleibt (siehe insbesondere Fig. 5b), d.h. sie muss nicht notwendiger Weise dem Kappenumfang folgen. Darüber hinaus kann die Grundrissform der Lasche dann weitestgehend beliebig gestaltet werden, d.h. sie kann sich gemäß der Fig. 5c in Richtung Wurzel verdicken und/oder verbreitern, um so eine größere Steifigkeit zu erhalten. Auch die Laschenwurzel selbst kann nach statischen Gesichtspunkten zur Erreichung einer möglichst hohen Steifigkeit frei dimensioniert und gestaltet werden.

Unabhängig davon, nach welcher Fertigungsvariante die Lasche 9 letztlich gebildet wird, erstreckt sie sich erfindungsgemäß vom den Anschlag 8 darstellenden Nutengrund in Richtung zur distalen Stirnseite des Endoskopkopfs 2 bzw. der Kappe 1, wobei deren abgerundete freie Vorderkante gegenüber der distalen Vorderkante der Spreizhülse 3 axial geringfügig zurückgesetzt ist.

Wie insbesondere aus den Fig. 2, 3 und 5 zu erkennen ist, ersteckt sich die Stirnnut 7 nicht exakt parallel zur Endoskop- bzw. Kappenmittelachse sondern ist in Richtung distaler Stirnseite zur Mittelachse hin geneigt. Darüber hinaus ist die Nut 7 nicht geradlinig sondern deren Nutenwände, zumindest die äußere Nutenwand, sind in Axialrichtung leicht gekrümmt, derart, dass sich die Nut 7 in ihrem axialen Mittenabschnitt radial nach Außen wölbt.

In einem axial vorderen Endabschnitt der Lasche 9 ist diese mit einer radialen äußeren Durchgangsbohrung 10 versehen, durch die ein Faden 11 oder Gewebe vom Nuteninneren in Richtung nach Außen der Kappe 1 geführt und darin fixiert ist. Vorzugsweise ist das eine Fadenende zu diesem Zweck an der Kappenwandaußenseite verknotet, sodass ein Zurückziehen des Fadens 11 durch die äußere Durchgangsbohrung 10 verhindert wird. Des Weiteren ist die Endoskopkappe 1 an einer Stelle im Wesentlichen radial gegenüberliegend zur vorstehend genannten Durchgangsbohrung 10, d.h. im Bereich der axial überstehenden Spreizhülse 3 mit einer radialen inneren Durchgangsbohrung 12 versehen, durch welche der Faden 11 vom Nuteninneren nach Innen geführt ist.

Wie insbesondere aus der Fig. 3 zu entnehmen ist, befindet sich die innere Durchgangsbohrung 12 axial unmittelbar vor der distalen Stirnseite des Endoskopkopfs 2, sodass der Faden 11 aus der inneren Durchgangsbohrung 12 kommend in einen an der Kopfstirnseite sich öffnenden Funktionskanal oder den Arbeitskanal des Endoskops einfädelbar ist, ohne dass dieser eine lange freie Wegstrecke zurücklegen muss.

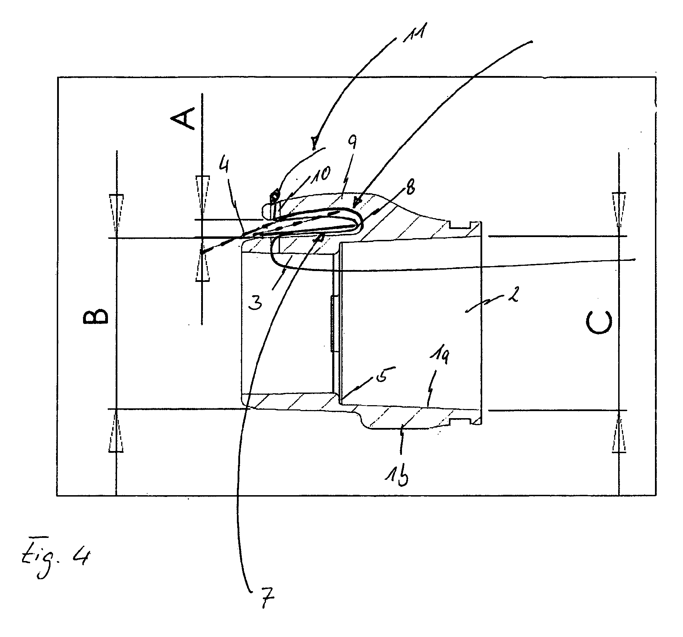

Die Arbeitsweise der erfindungsgemäßen Endoskopkappe mit der Halte- und Abziehfunktion wird nachfolgend insbesondere unter Bezugnahme auf die Fig. 4 näher beschrieben.

Um einen Gewebeclip 4 beispielsweise gemäß der Fig. 1 an seine vorbestimmte Position zu bringen, muss dieser zuerst auf die Spreizhülse 3 der Endoskopkappe 1 aufgezogen werden. Hierfür werden Unter- und Oberkiefer des Gewebeclips 4 von Hand aufgeklappt, sodass der Clip 4 an der gerundeten Vorderkante der Spreizhülse 3 angesetzt und über diese geschoben werden kann. Dabei dringt die Hinderkante des Gewebeclips 4 in die Stirnnut 7 der Endoskopkappe 1 ein und zieht dabei den Faden 11 aus dem Funktions- oder Arbeitskanal des Endoskopschafts 6 heraus.

Schließlich kommt die Schiebewegung des Clips 4 mit dessen in Anlage kommen am Nutengrund 8 zum Stillstand, wobei der Clip 4 sowie der mitgenommene Faden 11 die in Fig. 2 gezeigte Raumlage einnehmen. D.h. in dieser Lage ist der Clip 4 vollständig auf die Endoskopkappe 1 aufgezogen und kann so über das Endoskop in ein Hohlorgan eingebracht werden. Der Faden 11 umgreift dabei die Hinterkante des Clips 4 und erhält somit eine U-Form in Fadenlängsrichtung gesehen.

Soll der Clip 4 nunmehr abgestreift werden, wird der Faden 11, welcher durch den Schaftkanal bis zum proximalen Ende des Endoskops geführt ist, gezogen, wobei sich der die Stirnnut 7 in Radialrichtung durchquerende Fadenabschnitt verkürzt. Da der Faden 11 in der äußeren Durchgangsbohrung 10 fixiert ist, übt dieser mit einer entsprechenden Übersetzung nach dem Flaschenzugprinzip auf den Clip 4 eine Kraft in Axialrichtung aus, wodurch der Clip 4 in Richtung distales Ende der Endoskopkappe 1 verschoben wird. Durch die äußere Abrundung der vorderen Spreizhülsenkante sowie die weiche, d.h. gewölbte Formgebung der Stirnnut 7 wird ein Abgleiten des Clips 4 über die Vorderkante der Spreizhülse 3 erleichtert und die über den Faden 11 aufzubringende maximale Verschiebkraft weiter reduziert. Sobald die Hinterkante des Clips 4 aus der Stirnnut 7 getreten ist und daher nicht mehr von der Lasche 9 gehalten werden kann, bewirkt die im Clip 4 gespeicherte Vorspannkraft ein Abspringen des Clips 4 von der Spreizhülse 3. Damit ist der Abziehvorgang beendet und das Endoskop kann aus dem Hohlorgan genommen werden.

## Patentansprüche

1. Endoskopkappe (1) mit Halte - und Abzieheinrichtung für einen Gewebeclip (4), der auf eine einen Kappenmantelwand aufweisenden Spreizhülse (3) der Endoskopkappe (4) aufschiebbar ist, wobei die Endoskopkappe diese Spreizhülse (3) sowie einen an einer radialen Innenseite der Endoskopkappe verschiebbar, in einen Endoskopkanal für dessen Betätigung eingeführte oder einführbare Abziehfaden oder Gewebe (11) aufweist; **dadurch gekennzeichnet,**
**daß** die Endoskopkappe (1) eine Lasche (9), welche sich im Radialabstand zur Kappenmantelwand längs zur Endoskopkappe erstreckt und zwischen sich und der Kappenmantelwand eine an der Vorderkante der Spreizhülse (3) sich öffnende Stirnnut (7) ausbildet aufweist, sowie daß der Abziehfaden (oder Gewebe) in einem axial vorderen Kappenabschnitt die Stirnnut (7) radial durchquert.

2. Endoskopkappe nach Anspruch 1, **dadurch gekennzeichnet, dass** der Faden (11) an einer durch die Stirnnut (7) sich ausbildenen, coaxial sich erstreckenden Lasche (9) der Endoskopkappe radial außenseitig fixiert ist.

3. Endoskopkappe nach Anspruch2, **dadurch gekennzeichnet, dass** die Lasche (9) an ihrem freien vorderen Ende mit einer äußeren Radialbohrung (10) versehen ist, durch die der Faden (11) geführt und daran verankert ist, wobei radial gegenüberliegend an der Spreizhülse (3) eine innere Radialdurchgangsbohrung (12) ausgebildet ist, durch die der Faden (11) nach Durchqueren der Stirnnut (7) verschiebbar geführt ist.

4. Endoskopkappe nach einem der vorstehenden Ansprüche, **dadurch gekennzeichnet, dass** die Endoskopkappe einen Aufsteckabschnitt (1a) hat, der auf das distale Ende eines Endoskopschafts aufsteck-oder aufschraubbar ist, an den sich im Axialabstand die Spreizhülse (3) einstückig anschließt.

5. Endoskopkappe nach Anspruch4, **dadurch gekennzeichnet, dass** der Faden (11) axial vor dem Aufsteckabschnitt (1a) aus der Stirnnut (7) tritt.

6. Endoskopkappe nach einem der vorstehenden Ansprüche 4 oder 5, **gekennzeichnet durch** einen radial inneren Umfangsabsatz (5) als axiale Begrenzung des Aufsteckabschnitts (1a) und als Axialanschlag für einen eingesteckten oder eingeschraubten Endoskopschaft.

7. Endoskopkappe nach einem der vorstehenden Ansprüche, **dadurch gekennzeichnet, dass** die Stirnnut (7) in Richtung vordere Kante der Kappe zur Kappenmittelachse hin geneigt ist und/oder in Längsrichtung der Kappe radial nach Außen gewölbt ist.

8. Endoskopkappe nach einem der vorstehenden Ansprüche, **gekennzeichnet durch** eine äußere radiale Verdickung oder Wulst (1b) an der Außenseite der Kappenmantelwand, welche sich ausgehend vom Axialbereich des Nutengrunds (8) in Axialrichtung nach Hinten erstreckt und im Wesentlichen um den gesamten Umfang der Kappe geführt ist.

## Claims

1. An endoscope cap (1) having a holding and withdrawing means for a tissue clip (4) adapted to be slipped onto an expanding sleeve (3) of the endoscope cap (1) that comprises a cap sheath wall, wherein the endoscope cap comprises the said expanding sleeve (3) as well as a withdrawing thread or tissue (11) movably introduced or adapted to be movably introduced into an endoscope channel for operating the same at a radial inner face of the endoscope cap; **characterized in that**
the endoscope cap (1) comprises a tab (9) which extends longitudinally with respect to the endoscope cap (1) at a radial distance from the cap sheath wall, and between itself and the cap sheath wall forms a front groove (7) opening at the front edge of the expanding sleeve (3), and **in that** the withdrawing thread (or tissue) radially crosses the front groove (7) at an axial front cap portion (1).

2. The endoscope cap according to claim 1, **characterized in that** the thread (11) is fixed radially on the outside at a coaxially extending tab (9) of the endoscope cap formed by the front groove (7).

3. The endoscope cap according to claim 2, **characterized in that** the tab (9) is provided at its free front end with an outer radial bore (10) through which the thread (11) is guided and anchored to the same, wherein radially opposed thereto at the expanding sleeve (3) an inner radial through bore (12) is formed through which the thread (11) is movably guided after crossing the front groove (7).

4. The endoscope cap according to any one of the preceding claims, **characterized in that** the endoscope cap includes a slip-on portion (1a) adapted to be slipped or screwed onto the distal end of an endoscope shaft to which the expanding sleeve (3) is integrally connected at an axial distance.

5. The endoscope cap according to claim 4, **characterized in that** the thread (11) emerges from the front groove (7) axially ahead of the slip-on portion (1a).

6. The endoscope cap according to any one of the preceding claims 4 or 5, **characterized by** a radially inner circumferential shoulder (5) as an axial limitation of the slip-on portion (1a) and as an axial stop for a plugged-in or screwed-in endoscope shaft.

7. The endoscope cap according to any one of the preceding claims, **characterized in that** the front groove (7) is inclined in the direction of the front edge of the cap toward the central axis of the cap and/or is arched radially outwardly in the longitudinal direction of the cap.

8. The endoscope cap according to any one of the preceding claims, **characterized by** an outer radial thickening or bead (1b) at the outside of the cap sheath wall, which extends backwards in an axial direction starting from the axial area of the groove bottom (8) and is substantially guided around the entire circumference of the cap.

## Revendications

1. Bouchon pour endoscope (1) comportant un dispositif de maintien et de retrait pour une agrafe tissulaire (4) qui peut être poussé sur une gaine d'écartement (3) présentant une paroi de revêtement de bouchon du bouchon d'endoscope (4), dans lequel
le bouchon d'endoscope présente cette gaine d'écartement (3) et un fil de retrait (ou un tissu) (11) pouvant être décalé sur un côté intérieur radial du bouchon d'endoscope, introduit ou pouvant être introduit dans un canal d'endoscope pour son actionnement ; **caractérisé en ce que**
le bouchon d'endoscope (1) présente une languette (9) qui s'étend longitudinalement à distance radiale de la paroi de revêtement de bouchon par rapport au bouchon d'endoscope et présente, formée entre elle et la paroi de revêtement de bouchon, une rainure frontale (7) s'ouvrant sur le bord avant de la gaine d'écartement (3),
**en ce que** le fil de retrait (ou le tissu) croise radialement la rainure frontale (7) dans un tronçon de bouchon axial avant.

2. Bouchon d'endoscope selon la revendication 1, **caractérisé en ce que** le fil (11) est fixé radialement du côté extérieur à une languette (9) s'étendant co-axialement, se formant par la rainure frontale (7) du bouchon d'endoscope.

3. Bouchon d'endoscope selon la revendication 2, **caractérisé en ce que** la languette (9) est dotée sur l'une de ses extrémités avant libres, d'un alésage radial externe (10) au travers duquel le fil (11) est introduit et est ancré à celui-ci, un alésage de passage radial (12) étant formé du côté radialement opposé de la gaine d'écartement (3) au travers duquel le fil (11) est conduit de façon à pouvoir être décalé, après avoir traversé la rainure frontale (7).

4. Bouchon d'endoscope selon l'une des revendications précédentes, **caractérisé en ce que** le bouchon d'endoscope a un segment d'empilement (la) qui peut être empilé ou vissé sur l'extrémité distale d'une tige d'endoscope à laquelle est raccordée d'un seul tenant à distance axiale, la gaine d'écartement (3).

5. Bouchon d'endoscope selon la revendication 4, **caractérisé en ce que** le fil (11) sort axialement devant le segment d'empilement (la) de la rainure frontale (7).

6. Bouchon d'endoscope selon l'une des revendications précédentes 4 ou 5, **caractérisé par** un épaulement périphérique intérieur radial (5) comme limite axiale du segment d'empilement (la) et comme butée axiale d'une tige d'endoscope enfichée ou vissée.

7. Bouchon d'endoscope selon l'une des revendications précédentes, **caractérisé en ce que** la rainure frontale (7) est orientée dans la direction du bord avant du bouchon vers l'axe médian du bouchon et/ou est courbée radialement vers l'extérieur dans la direction longitudinale du bouchon.

8. Bouchon d'endoscope selon l'une des revendications précédentes, **caractérisé par** un épaississement ou un bourrelet (1b) radial extérieur sur le côté extérieur de la paroi de revêtement de bouchon qui s'étend à partir de la zone axiale du fond de la rainure (8) en direction axiale vers l'arrière et évolue essentiellement sur le pourtour complet du bouchon.
